Europäisches Patentamt

European Patent Office

Office européen des brevets

(11) Publication number: **0 269 455**
**A2**

(12) # EUROPEAN PATENT APPLICATION

(21) Application number: **87310475.6**

(22) Date of filing: **27.11.87**

(51) Int. Cl.⁴: **C 12 N 15/00**
**C 07 K 15/06, C 12 P 21/00**

(30) Priority: **28.11.86 JP 281871/86**
**18.09.87 JP 232295/87**

(43) Date of publication of application:
**01.06.88 Bulletin 88/22**

(84) Designated Contracting States:
**AT BE CH DE ES FR GB GR IT LI LU NL SE**

(71) Applicant: **Takeda Chemical Industries, Ltd.**
**27, Doshomachi 2-chome Higashi-ku**
**Osaka-shi Osaka, 541 (JP)**

(72) Inventor: **Ikeyama, Shuichi**
**32-15, Nanpeidai 5-chome**
**Takatsuki§Osaka 569 (JP)**

**Nishimura, Osamu**
**54-16, Daiwanishi 1-chome**
**Kawanishi Hyogo 666-01 (JP)**

(74) Representative: **Laredo, Jack Joseph et al**
**Elkington and Fife High Holborn House 52/54 High**
**Holborn**
**London, WC1V 6SH (GB)**

The applicant has filed a statement in accordance with Rule 28 (4) EPC (issue of a sample only to an expert). Accession numbers of the deposits: IFO - 50056; KFCC - 10280.

(54) **Highly purified fused protein comprising human IgE Fc fragment and production thereof.**

(57) A highly purified fused protein which comprises a first protein having the amino acid sequence:
Ala-Pro-Thr-Ser-Ser-Ser-Thr-Lys-Lys-Thr-Gln-Arg-Asn-Ser-Met-Leu and a human IgE Fc fragment joined to the C-terminus of the first protein, and a production method thereof comprising the purifying procedure comprising immunoaffinity chromatography. The fused protein is useful for allergy medicine.

EP 0 269 455 A2

**Description**

HIGHLY PURIFIED FUSED PROTEIN COMPRISING HUMAN IgE Fc FRAGMENT AND PRODUCTION
THEREOF

Background of the Invention

(a) Field of the Invention
The present invention relates to a fused protein comprising human IgE (human immunoglobulin E) Fc fragment which is useful as a medicine for allergy, and to a production method thereof.

(b) Description of the Prior Art
IgE is a kind of immunoglobulin Ig. Ig has basically a Y-letter structure. IgE is usually bound to the Fc receptor located on the membranes of mast cells and basophils at the Fc fragment thereof which corresponds to the stem of the Y-structure. Reaction of an Fab fragment which corresponds to the branch of the Y-structure against antibodies makes the cells release such biological active materials as histamine to induce acute allergy.

The following mechanism of the inhibition of releasing histamine is known:
An Fc fragment which consists of only Fc part (consisting of CH2, CH3 and CH4) has ability to bind to the Fc receptor, but it does not react against antibodies because of lack of Fab part. As a result, an Fc fragment competes with the complete IgE having Fab part and Fc part in the binding reaction to the Fc receptor. The competition inhibits the cells from releasing histamine.

Human IgE to be a raw material for an Fc fragment exists only in a very small amount in normal human serum. So human IgE is prepared from serum of myeloma patients containing high concentration of IgE. Accordingly it is impossible to obtain the desired amount of IgE under the same conditions, when necessary.

Some of the present inventors succeeded in obtaining highly purified human IgE from the supernatant of the cell lined human myeloma cell culture (See Japanese Unexamined Patent Publication No. 96028/1983). However, it is very difficult to obtain an Fc fragment by digesting IgE with proteinases. So the method provides only little Fc fragment and the method remains impractical.

Recent Progress in molecular biology has made it possible to introduce a DNA sequence coding an Fc fragment into bacteria to express it. Expression of an Fc fragment-DNA in bacteria by gene engineering techniques is expected to give the possibility of mass production of the Fc fragments and the possibility of their practical usage as an allergic medicine.

The nucleotide sequence of IgE gene of myeloma cells taken from a myeloma patient has been determined [Proc. Natl. Acad. Sci. U.S.A. 79 6661(1982), Nucleic Acids Res. 11 719 (1983)], and expression of the Fc fragment in E. coli has been reported [Nucleic Acids Res. 11 3077, Proc. Natl. Acad. Sci. U.S.A. 81 2955(1984)]. However, as an Fc fragment has nine cysteine residues, an Fc fragment protein which is extracted from E. coli under reducing conditions does not have a native tertiary structure. The technique of constructing the native tertiary structure of the produced protein by recombinant DNA techniques has not yet been established, and it is a problem to be solved.

Expression of Fc fragment DNA in animal cells and secretion thereof out of the cells makes it possible to prepare the Fc fragment with native tertiary structure in large quantities. Recently a technique has been disclosed using an IL-2 leader sequence which allows animal cells to express human IgE Fc fragment DNA and to accumlate an Fc fragment efficiently in a culture medium (Japanese Patent Application No. 182457/1986 corresponding to European Patent Publication No. 225,701 published on June 16, 1987). However, in the above case, only Fc fragment-activity was detected in the culture supernatant, but an Fc fragment with native tertiary structure could not be isolated. Accordingly, development of an efficient isolating- and purifying- technique for an Fc fragment accumulated in the medium is expected to give an Fc fragment with native tertiary structure in large quantities and to contribute to the progress of allergic therapy.

Summary of the Invention
The present inventors have been studying the efficient isolation and purification method of the accumulated Fc fragment in the medium, and have found that the protein containing homogeneous human IgE Fc fragment may be obtained in high purity by the purification procedure comprising immunoaffinity chromatography.

Generally it is necessary to use the gene coding for mature protein in the form having a DNA (leader sequence) coding for the signal peptide to the protein as added to the 5' end thereof, in order to express the useful gene in animal cells and secrete the protein into the medium. However, in the case that the leader sequence is unknown or can not be obtained easily, a DNA fragment comprising a protein-encoding DNA sequence and a DNA sequence coding for a signal peptide of a different, animal cell-derived protein as joined to said protein-encoding DNA sequence on the upstream side thereof without causing any reading frame shift can be used (Japanese Patent Application No. 182457/1986).

In this invention, a human IgE Fc fragment is produced by the method of the above-described Japanese Application. Thus, the present invention embraces a method of producing a protein, which comprises cultivating in a medium an animal cell transformed with an expression vector for an animal cell, the expression vector having a DNA comprising a protein-encoding DNA sequence and a DNA sequence coding for a signal peptide of a different, animal cell-derived protein joined to said protein-encoding DNA sequence on the upstream side thereof without causing any reading frame shift as well as a promotor DNA capable of functioning in an animal cell on the upstream side of the DNA sequence coding for the signal peptide, and collecting the protein thus secreted and accumulated in the medium by the purifying procedure comprising immunoaffinity chromatography.

As a result, the highly purified fused protein comprises the first protein consisting of 11 amino acid residues corresponding to IL-2 N-terminal 11 residues: Ala-Pro-Thr-Ser-Ser-Ser-Thr-Lys-Lys-Thr-Gln, and linker: Arg-Asn-Ser-Met-Leu, and human IgE Fc fragment added to the C-terminal of the first protein. The above first protein may contain sugar. The fused protein is, as mentioned later, considered as homogeneous and pure protein from the result of SDS-PAGE and N-terminal amino acid sequence.

The invention relates to

(1) A highly purified fused protein which comprises the first protein having the amino acid sequence: Ala-Pro-Thr-Ser-Ser-Ser-Thr-Lys-Lys-Thr-Gln-Arg-Asn-Ser-Met-Leu and a human IgE Fc fragment joined to the C-terminus of the first protein; and

(2) A production method for the highly purified fused protein which comprises cultivating in a medium an animal cell capable of secreting a fused protein which comprises the first protein having the amino acid sequence: Ala-Pro-Thr-Ser-Ser-Ser-Thr-Lys-Lys-Thr-Gln-Arg-Asn-Ser-Met-Leu and a human IgE Fc fragment joined to the C-terminus of the first protein, and collecting the protein accumulated in the medium by the procedure comprising immunoaffinity chromatography. The fused protein according to the invention has the following properties:

(a) Specific activity is at least $5 \times 10^5$ units/mg,

(b) The molecular weight based on the SDS-poly acrylamide gel electrophoresis is 110,000 $\pm$ 10,000 dalton under non-reducing conditions, and 55,000 $\pm$ 5,000 dalton under reducing conditions, and

(c) It is a glycosylated protein comprising sugar.

The protein according to the invention includes the protein a portion of which is deleted, or the protein a portion of which is replaced with other amino acids, as long as they have the same function as the protein.

Brief Description of the Drawing

Fig. 1 shows the construction scheme of plasmid pTB543 which is used to obtain the fused protein.

Fig. 2 shows the construction scheme of plasmids pTB406, pTB410 and pTB411 which are used to obtain the fused protein.

Fig. 3 shows the nucleotide sequence of the leader sequence and structural gene of IL-2,

Fig. 4 shows the construction scheme of plasmid pTB503 which is used to obtain the fused protein.

Fig. 5 shows the amino acid sequence translated from the nucleotide sequence of IL-2 leader sequence, IL-2 structural gene portion, linker and IgE Fc portion which are included in plasmid pTB543.

Fig. 6 shows the result of SDS-polyacrylamide slab gel electrophoresis to the fused protein obtained according to the present invention.

Description of the Preferred Embodiment

As the animal cells which are capable of secreting the fused protein according to the invention, the following animal cells are preferably cited: The animal cell transformed with an expression vector for an animal cell, the expression vector having a DNA comprising a human IgE Fc fragment-encoding DNA sequence and a DNA sequence coding for a signal peptide of IL-2 as a different, animal cell-derived protein joined to said protein-encoding DNA sequence on the upstream side thereof without causing any reading frame shift as well as a promotor DNA capable of functioning in an animal cell on the upstream side of the DNA sequence coding for the signal peptide.

The DNA sequence coding for the signal peptide of a different, animal cell-derived protein is a DNA sequence coding for the signal peptide of an animal cell-derived protein different from the protein in relation to the above-mentioned gene and includes, among others, those DNA sequences coding for the signal peptides of human interleukin-2 (IL-2) (Japanese Unexamined Patent Publication No. 115528/1985, which corresponds to European Patent Publication No. 145390), IFN-γ (Japanese Unexamined Patent Publication open No. 189197/1983, which corresponds to European Patent Publication No. 89676), human tumor necrosis factor (TNF) [Nature, 312: 724 (1984)] and human lymphotoxin (LT) [Nature, 312: 721 (1984)].

A preferred example of such DNA sequence is a sequence coding for the signal peptide of IL-2 which has the formula:

Met Tyr Arg Met Gln Leu Leu Ser Cys Ile Ala Leu Ser Leu Ala Leu Val Thr Asn Ser　　(I)

and the leader sequence of IL-2 which has the formula

GCA CCT ACT TCA AGT TCT ACA AAG AAA ACA CAG CTA CAA CTG GAG CAT TTA CTG CTG GAT　　(II)

is particularly preferred.

The above-mentioned DNA sequence coding for the signal peptide of the protein may have, at the 3′ terminus thereof, a DNA sequence coding 1 to 20 amino acids of the N-terminal polypeptide of the protein.

The DNA sequence according to the invention which codes for the signal peptide of a different, animal cell-derived protein can be produced as a cDNA (leader sequence) synthesized based on an mRNA or as a DNA sequence derived chemically by half synthesis [Tetrahedron Letters, 22: 1859 (1981)] or total synthesis

3

using a per se known method, for example the phosphorous amide method.

In producing the leader sequence for a protein on the basis of an mRNA, a plasmid containing the leader sequence DNA for the protein can be produced, for example by

(a) isolating the mRNA coding for the animal cell-derived protein,

(b) synthesizing a single-stranded cDNA based on said RNA and then a double-stranded DNA,

(c) inserting said cDNA into a plasmid,

(d) transforming a host with the recombinant plasmid obtained,

(e) cultivating the transformant obtained, then isolating the plasmid containing the leader sequence for the protein from the transformant,

(f) cutting out the desired, cloned leader sequence DNA from the plasmid, and

(g) inserting the cloned DNA into a vehicle at a site downstream from a promoter in the vehicle.

This plasmid can be introduced into an appropriate host, for example Escherichia coli. From among the transformants obtained in this manner, the desired clone can be picked out by a per se known method, for example by colony hybridization. This series of fundamental procedures is known in the art and described in detail in Methods in Enzymology, 68 (1979) and Molecular Cloning (1982), Cold Spring Harbor Laboratory.

After producing a plasmid from the above transformant by a per se known method, the leader sequence DNA can be isolated by a per se known method, for example by cleaving the plasmid with a restriction enzyme, followed by polyacrylamide gel electrophoresis or agarose gel electrophoresis, for instance. The nucleotide sequence of the DNA fragment isolated can be determined by a per se known method, for example by the dinucleotide synthesis chain termination method [Proc. Natl. Acad. Sci. USA, 74: 5463 (1977)].

The promoter to be used in the practice of the invention which is capable of functioning in animal cells may be any promoter provided that it is competent and suited for the host to be used for gene expression. There may be mentioned, for example, the SV40-derived promoter and a retrovirus-derived promoter, which are suited for the expression in the monkey cell lines COS-7 and Vero, the chinese hamster cell line CHO and the mouse cell line L, among others.

A DNA fragment coding for a human IgE Fc fragment used in the invention includes the natural one obtained from myeloma patient serum or normal human serum, a whole synthesized one and half synthesized one, as long as they have human IgE Fc fragment activity.

The expression vector for animal cells according to the invention can be produced, for example by inserting a DNA comprising the protein-encoding DNA sequence and the DNA sequence coding for the signal peptide of a different, animal cell-derived protein joined to said protein-encoding DNA sequence on the upstream side thereof without causing any reading frame shift as well as the promoter capable of functioning in animal cells into a known vector, such as pBR322, using a restriction enzyme or enzymes and ligase to thereby construct said vector.

The transformed animal cells according to the invention can be produced by transforming animal cells with the above-mentioned expression vector for animal cells, as necessary followed by cloning or harvesting.

The above animal cells may be any cells capable of allowing expression of the leader sequence for the protein. Examples are mouse cells (e.g. L cells, Balb/3T3 cells), human cells (e.g. FL cells) and hamster cells (e.g. CHO cells).

The transformation is advantageously effected by cotransfection [Cell, 16: 777 (1979)], for instance, whereby the desired animal cell transformant can be cloned and isolated.

The cultivation of animal cells is conducted in a per se known medium for animal cell culture, for example in MEM medium containing about 5 to 20% of fetal calf serum, at about 30 to 40°C for about 1 to 10 days.

The IgE Fc fragment produced and accumulated in the medium can be isolated and purified from the supernatant obtained by centrifugation of the culture by an appropriate combination of per se known separation and purification methods.

Thus, for example, it may be one or a combination of the methods, ammonium sulfate salting out, immunoaffinity chromatography using monoclonal antibody and gel filtration chromatography. The most preferable method is the combination thereof in the above-described order.

Ammonium sulfate salting out is carried out in the usual procedure, for example, the fraction precipitated under saturation with 40-60% ammonium sulfate is collected by centrifugation, and solubilized into phosphate-buffered saline(PBS), followed by dialysis against the buffer for 20-40 hours. It is preferable to exchange the buffer in the middle of the procedure. These procedures are usually carried out at low temperature (0 to 10°C).

Anti human IgE polyclonal antibodies may be used as a ligand of immuno-affinity chromatography, and among those the most preferable one is a monoclonal antibody recognizing Fc portion.

A monoclonal antibody recognizing Fc portion includes E235I63, E120.02 [Hybridoma 4, 47(1985)].

A carrier for immunoaffinity chromatography includes Affi Gel 10(Bio-rad), CNBr-activated Sepharose 4B(Pharmacia), and Formyl Cellulofine (Seikagaku Kogyo), among which Affi Gel 10 is preferable.

An Fc fragment is purified by immunoaffinity chromatography using E235I63 as a ligand as follows: The above-described Fc fragment containing solution (ammonium sulfate fraction) is adsorbed to the above-described equilibrium column with buffer such as PBS, followed by elution with the buffer. An appropriate amount of additives, for example a protein denaturator such as urea, may be added to the buffer. An appropriate eluation is prepared by the combination of kind of additives, concentration of additives, and pH of the buffer. A ultra filtration membrane and so on may be used for the concentration of the obtained fractions containing the Fc fragment.

Gel filtration chromatography is carried out by known procedure, for example using sephacryl S-200 column (Pharmacia).

The measurement of purity of the thusly obtained human IgE Fc fragment(specific activity) was carried out by a kit for human IgE measurement on the market, for example, IgE RIA Shionogi® (Shionogi).

The fused protein containing homogeneous and pure human IgE Fc fragment which is highly purified and available for medicines, is obtained by the production method according to the invention. This protein which is obtained by purifying the fused protein which is produced by an animal cell transformed with DNA having a nucleotide sequence coding for a human IgE Fc fragment according to the method of the invention has the following properties:

(1) It is homogeneous in SDS-poly acrylamide gel electrophoresis and the measured molecular weight (under non-reducing conditions) by the method is $110,000 \pm 10,000$ dalton.

(2) Dimer is formed by disulfide bonds and the measured molecular weight is changed to $55,000 \pm 5,000$ dalton by reduction.

(3) It has alanine as an N-terminal amino acid.

(4) It has lysine as a C-terminal amino acid.

(5) It is a glycosylated protein which contains sugar, and

(6) It has specific activity of at least $5 \times 10^5$ units/mg.

It is considered that the protein obtained according to the invention is substantially homogeneous from the above items (1), (3) and (4) and that the protein retains tertiary structure from the above items (6) and (2).

The measurement of the activity (specific activity) of the fused protein containing an Fc fragment for the determination of protein purity is carried out by radioimmunoassay using IgE RIA Shionogi kit.

Toxicity of the fused protein of the invention is low and the protein has a therapeutic function for allergy induced by IgE. Accordingly, the fused protein of the invention may be used, for example, for therapeutic medicine for allergy such as allergic dermatosis, atopic dermatitis and bronchial asthma. The protein according to the invention is percutanously dosed by spraying or applying of spray liquid, ointment and cream or orally dosed in a tablet. Dosage amount depends on properties or dosage method, with general dosages being about 10ng-10μg/dosage and about 3 dosages/day.

In the present specification and the accompanying drawings, the nucleotides and the amino acids, when given in terms of abbreviations, are abbreviated in accordance with the recommendations of the IUPAC-IUB Commission on Biochemical Nomenclature or the practice conventional in the relevant field of art. The following is a listing of several abbreviations. In case of amino acids having optical isomers, the L-form is intended except as indicated.

DNA: Deoxyribonucleic acid
A: Adenine
T: Thymine
G: Guanine
C: Cytosine
SDS: Sodium dodecylsulfate
Gly: Glycine
Ala: Alanine
Val: Valine
Leu: Leucine
Ile: Isoleucine
Ser: Serine
Thr: Threonine
Cys: Cysteine
1/2Cys: Half cystine
Met: Methionine
Glu: Glutamic acid
Asp: Asparatic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
cDNA: Complementary deoxyribonucleic acid
RNA: Ribonucleic acid

Example

The present invention will be described in more detail hereinafter by way of examples. These examples, however, are not to be construed as limiting. the present invention.

Animal cell line, mouse L-IS11 IgE-9, obtained in Reference Example 1 has been deposited at IFO under the accession number IFO-50056 since July 18, 1985 and at KFCC (Korea) under the accession number KFCC-10280 since September 26, 1986.

Reference Example 1

Expression vectors for secretion of human IgE and animal cell transformants capable of producing human IgE

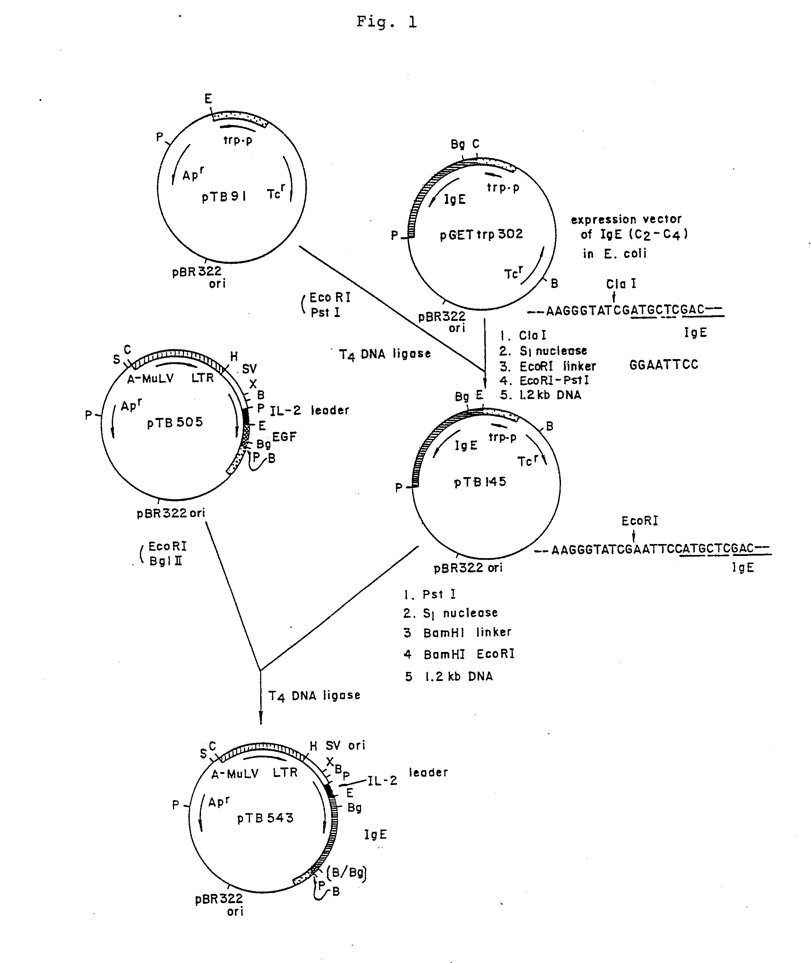

(i) Construction of plasmids pTB541, pTB542, pTB543 and pTB544 (Fig. 1)

(i)-1 The plasmid pGETtrp302 constructed such that the $C_2$-$C_4$ regions of the human immunoglobulin $\varepsilon$ chain (IgE) can be expressed in Escherichia coli [Kurokawa et al., Nucleic Acids Res., 11: 3077-3085 (1983)] was cleaved at the ClaI site occuring between the trp promoter and the translation initiation codon ATG and, after rendering the cohesive ends blunt by $S_1$ nuclease treatment, joined with the EcoRI linker GGAATTCC. After cleavage of the product with EcoRI and PstI, a 1.2 kb DNA fragment containing the IgE-encoding sequence was isolated. This 1.2 kb EcoRI-PstI DNA fragment was mixed with the EcoRI-PstI-cleaved plasmid pTB91 [Example 5 (ii) of Japanese Unexamined Patent Publication No. 88881/1986 which corresponds to European Patent Publication No. 177915] and from them pTB145 was constructed by T4 DNA ligase reaction. pTB145 was cleaved with PstI, the cohesive ends were rendered blunt with $S_1$ nuclease, the BamHI linker CCGGATCCGG was ligated to the cleavage product, and the resulting product was digested by EcoRI-BamHI digestion to give a 1.2 kb EcoRI-BamHI DNA fragment.

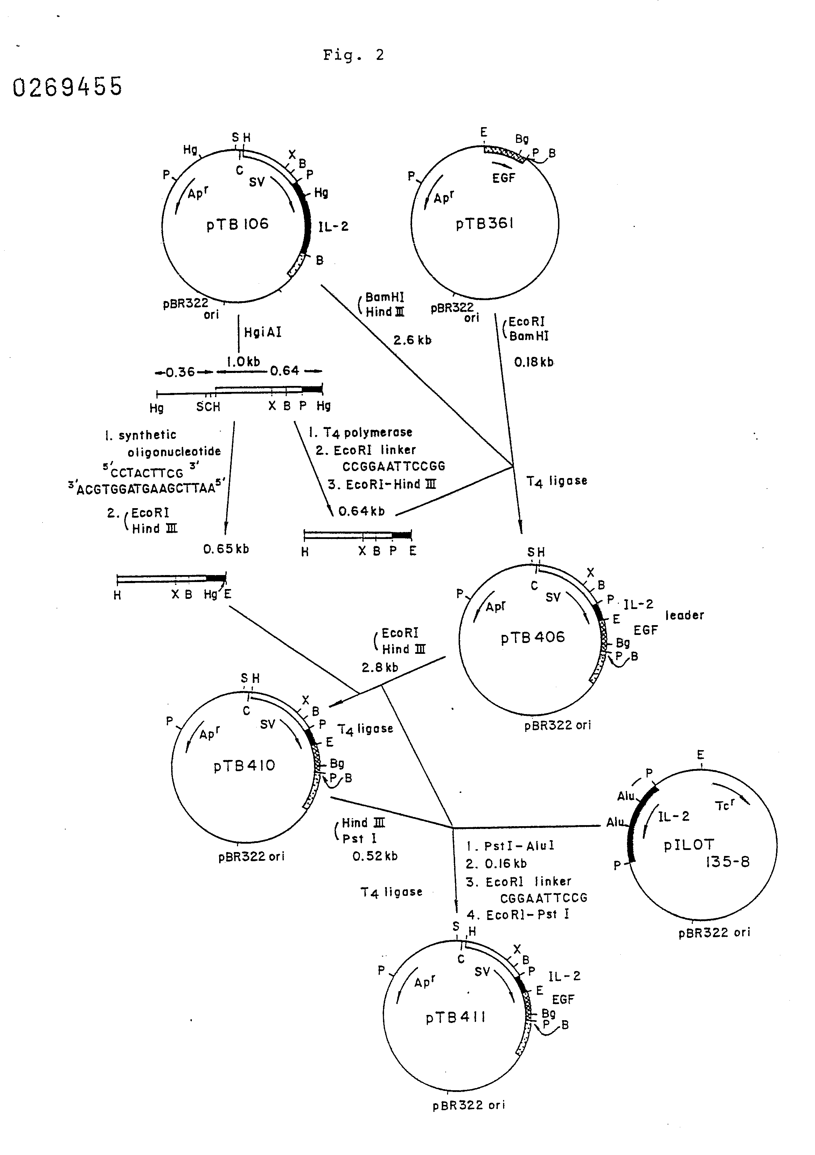

(i)-2 Construction of the plasmids pTB406, pTB410 and pTB411 (Fig.2)

The IL-2 gene expression vector pTB106 for animal cells [Example 1-(i) of Japanese Unexamined Patent Publication No. 63282/86 which corresponds to European Patent Publication No. 172619] was cleaved with the restriction enzyme HgiAI and a 1.0 kb DNA fragment containing the IL-2 gene leader sequence was isolated. The cohesive ends were made blunt by T4 DNA polymerase reaction. Following addition of the EcoRI linker CCGGAATTCCGG to the blunt end using T4 DNA ligase, complete digestion with EcoRI and HindIII was carried out and a 0.64 kb DNA fragment comprising an SV40 DNA-derived sequence (promoter and splicing site) and the IL-2 gene leader sequence was isolated.

The above pTB106 was cleaved with BamHI and HindIII and a 2.6 kb DNA fragment comprising the DNA replication origin for the replication of pBR322-derived plasmids in Escherichia coli, a sequence containing the ampicillin resistance gene, and the SV40 DNA-derived poly-A addition site, was isolated.

A 0.18 kb EcoRI-BamHI DNA fragment coding for human EGF was produced from pTB361 [Example 4 of Japanese Unexamined Patent Publication No. 88881/1986, which corresponds to European Patent Publication No. 177915) in which a synthetic human EGF gene has been cloned.

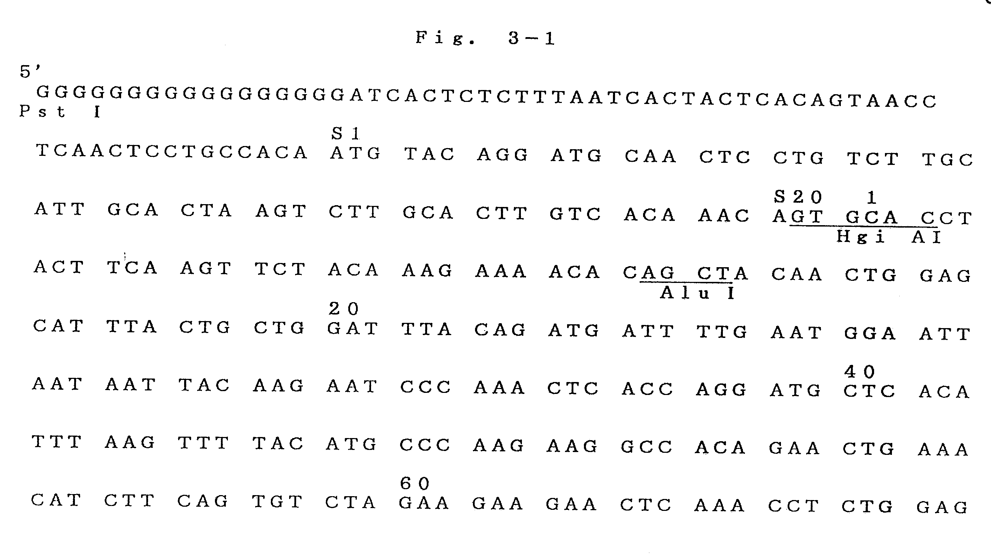

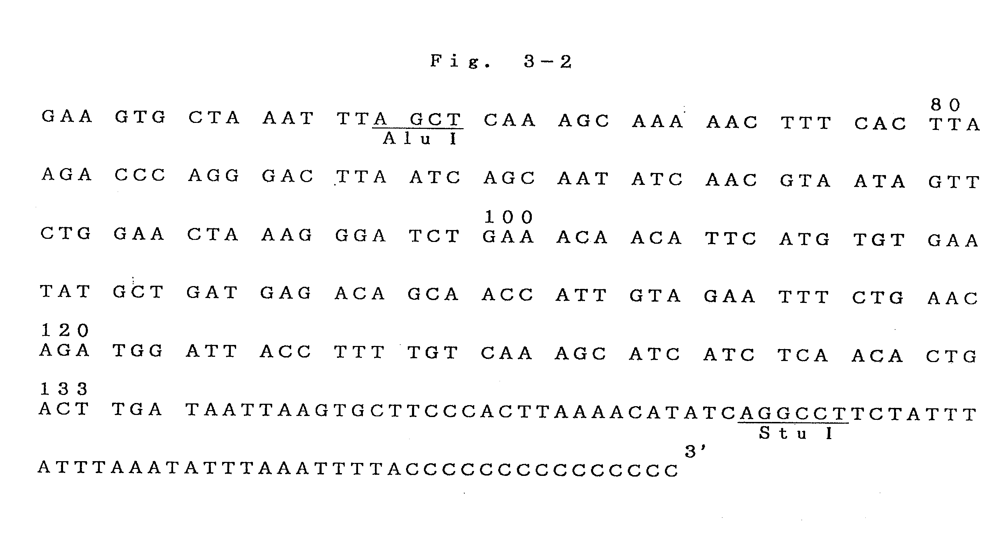

The above three DNA fragments were ligated together by T4 DNA ligase reaction to thereby construct pTB406. pTB406 has a structure comprising the leader sequence covering No. 1-123 nucelotides (Fig. 3) of the IL-2 gene DNA and the synthetic human EGF gene DNA as joined together by the intermediary of the EcoRI linker and the translation initiation codon ATG of the EGF DNA as inserted without causing any reading frame shift.

Then, the above-mentioned 1.0 kb DNA fragment obtained by cleavage of the plasmid pTB106 with HgiAI was mixed with the synthetic oligonucleotides 5′CCTACTTCG3′ and 5′AATTCGAAGTAGGTGCA3′ each produced by a known method, the mixture was subjected to reaction in the presence of T4 DNA ligase and the ligation product was cleaved with EcoRI and HindIII. A 0.65 kb EcoRI-HindIII DNA fragment was then isolated and purified. The thus-obtained DNA fragment has an SV40 DNA-derived sequence, and the whole IL-2 gene leader sequence and the nucleotide sequence coding for the four amino acids on the N-terminal side of the IL-2 protein molecule. The previously constructed pTB406 was cleaved with EcoRI-HindIII and a 2.8 kb DNA fragment containing the EGF gene, the poly-A addition site and the pBR322-derived sequence was isolated. This fragment was ligated with the above-mentioned 0.65 kb EcoRI-HindIII DNA fragment to thereby construct pTB410.

Further, pILOT135-8 (Japanese Unexamined Patent Publication No. 115528/1985, which corresponds to European Patent Publication No. 145390) was cleaved with PstI and AluI and a 0.16 kb DNA fragment having the sequence coding for the IL-2 gene leader sequence and the 11 amino acids on the N-terminal side of the IL-2 molecule was isolated. The EcoRI linker CGGAATTCCG was added to the blunt end resulting from AluI cleavage to construct a DNA fragment. This 0.16 kb fragment was ligated with the above-mentioned 2.8 kb DNA fragment obtained by EcoRI-HindIII cleavage of pTB406 and a 0.52 kb DNA fragment obtained by HindIII-PstI cleavage of pTB410 by T4 DNA ligase reaction to thereby construct pTB411.

The construction scheme for these EGF expression vectors, pTB406, pTB410 and pTB411 were shown in Fig. 2.

(i)-3 Construction of plasmids pTB503, pTB504 and pTB505

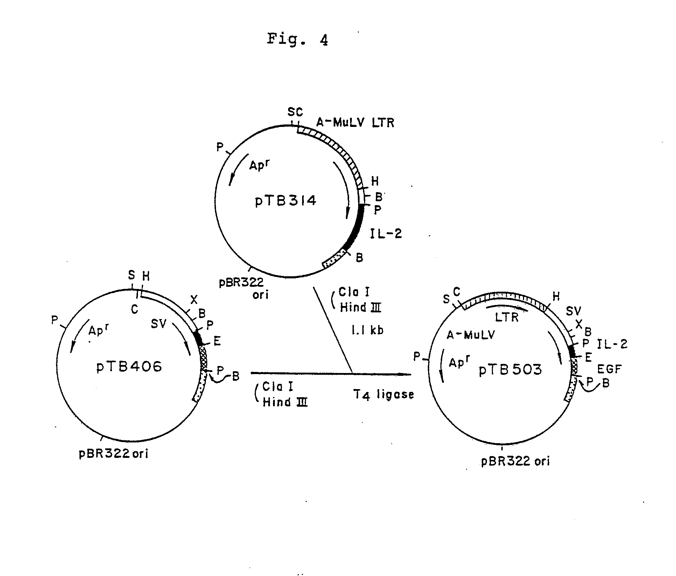

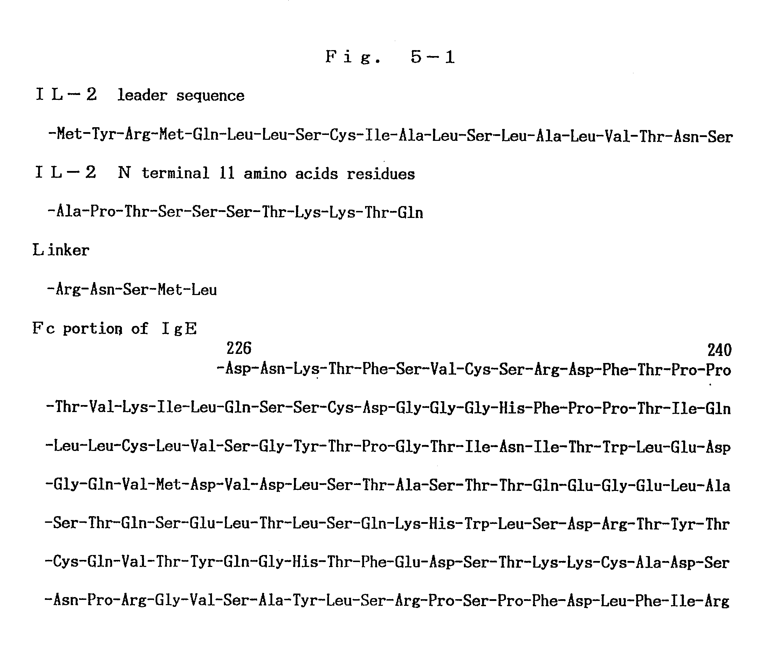

The human IL-2 leader sequence-containing human EGF expression vectors obtained in the above (i)-2, namely pTB406, pTB410 and pTB411, were respectively cleaved at the ClaI and HindIII sites occurring upstream from the SV40 promoter, followed by insertion of a 1.1 kb ClaI-HindIII DNA fragment separated and purified from pTB314 [Example 1 (iii) of Japanese Unexamined Patent Publication No. 63282/86 which corresponds to European Patent Publication No. 172619] and containing the Abelson mouse leukemia virus (A-MuLV) LTR region. Thus were constructed pTB503, pTB504 and pTB505, respectively. These recombinants are expected to bring about high-efficiency expression of the gene as desired particularly in mouse cells as compared with the case of only SV-40 promoter. The construction scheme for pTB503 is shown in Fig. 4 by way of example.

(i)-4 The plasmid pTB505 previously described in (i)-3 was cleaved with EcoRI-BgIII to thereby remove a major portion of the EGF-encoding sequence and, in lieu of this portion, the 1.2 kb EcoRI-BamHI DNA fragment previously prepared from pTB145 and having the sequence coding for the $C_2$-$C_4$ region polypeptide of IgE was inserted. Thus was constructed pTB543. Thus, pTB543 is a recombinant plasmid having the IL-2 leader sequence, the sequence coding for the 11 amino acids on the N-terminal side of IL-2, the IgE-encoding sequence joined to said sequence encoding the 11 amino acids without causing any reading frame shift and, upstream from these sequences, the A-MuLV LTR and SV40 promoter regions as the promoters for gene expression in animal cells (Fig. 1).

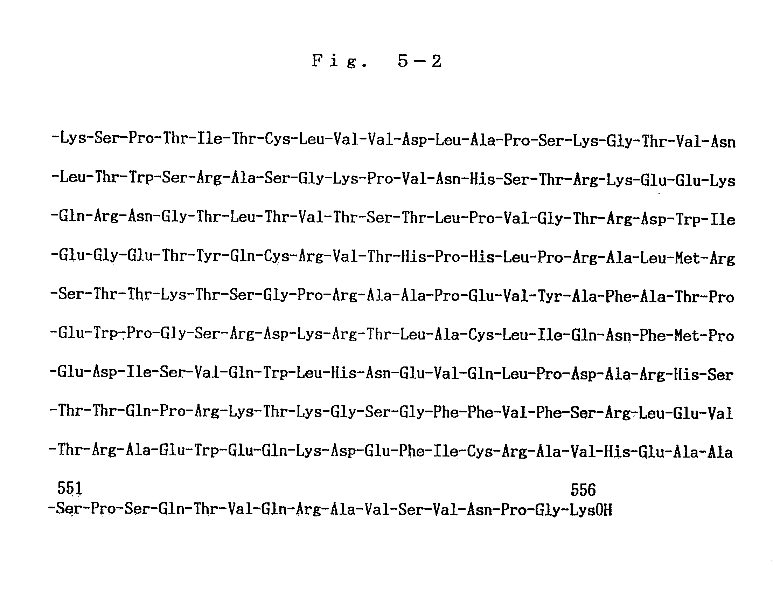

The amino acid sequences of IL-2 leader, N-terminal side of IL-2, linker and an IgEFc fragment are shown in Fig. 5.

(i)-5 In the same manner as above, pTB503 and pTB504 obtained in previously described in (i)-3 were each cleaved with EcoRI-BgIII, and pTB541 and pTB542 were constructed from the respective fragments and the above-mentioned pTB145-derived, IgE-encoding 1.2 kb BamHI-EcoRI DNA fragment. pTB541 has a structure such that the IL-2 leader sequence is joined to the IgE-encoding sequence and pTB542 has a structure such that the IL-2 leader sequence plus the sequence coding for the 4 amino acids on the N-terminal side of IL-2 is joined to the IgE-encoding sequence.

(i)-6 Furthermore, pTB544 having no IL-2 gene-derived leader sequence was constructed by replacing the EGF-encoding sequence in pTB506 described in Example 7 (i) in the specification for Japanese Patent Application No. 176976/1985 (filed August 13, 1985), which corresponds to European Patent Publication No. 177,915, with the pTB145-derived IgE-encoding sequence in the same manner as above.

(ii) Transformation of animal cells

Eagle's MEM medium containing 10% fetal calf serum was placed in Falcon dishes (6 cm in diameter) and mouse TK-deficient L cells were cultivated overnight at 37°C. Thereafter, these cells (7 × 10⁵ cells/dish) were inoculated with a mixture of 0.2 μg of the plasmid pTK61 [the product of re-cloning in pBR322 of a TK gene-containing 2 kb PvuII fragment (Proc. Natl. Acad. Sci. USA, 78: 1441-1445 (1981)) of a plasmid isolated from Escherichia coli LE578 (Gene, 7: 335-342 (1979); gift of Dr. Enquist) carrying a recombinant plasmid obtained by cloning in pBR322 of a 3.5 kb BamHI DNA fragment containing the herpes simplex virus (HSV) TK gene] and 10 μg of one of the pTB541, pTB542, pTB543 and pTB544 DNAs according to the method of Graham et al. [Virology, 52: 456-467 (1973)]. After 4 hours of cultivation at 37°C, the medium was replaced with a fresh portion and cultivation was conducted overnight. On the next day, the medium was replaced with HAT medium (MEM medium containing 15 μg/ml hypoxanthine, 1 μg/ml aminopterine, 5 μg/ml thymidine and 0.25 μg/ml glycine) containing 10% fetal calf serum and cultivation was continued at 37°C.

The cultivation was continued with medium exchange at 3- to 4-day intervals. Multiplication of those cells which had become TK+ resulted in colony formation in about 2 to 3 weeks.

(iii) Cloning of the transformants

The transformed cells obtained in Reference Example 1 (ii) were cloned by the limited dilution method. According to the method, cells L-ISO IgE-4 and L-ISO IgE-6 transformed with the plasmid pTB541, cells L-IS4 IgE-7 and L-IS4 IgE-10 transformed with the plasmid pTB542, cells L-IS11 IgE-3 and L-IS11 IgE-9 (IFO-50056, KFCC-10280) transformed pTB543, and cells L-IgE-1 and L-IgE-3 transformed with pTB544 were respectively obtained.

Reference Example 2

Preparation of E235I63-Affi Gel 10 column

A resin consisting of 25ml of Affi Gel 10® to which 150mg of anti-human IgE monoclonal antibody, E235I63, was bound [Molecular Immunology, 23, 159(1986)] was rinsed with PBS, 0.2M of sodium acetate buffer (pH 4.0) containing 0.15M of NaCl and 3M urea, and PBS in turn, and then the column with 1.5cm of an inner diameter was filled with them, thereby to prepare E235I63-Affi Gel 10 column.

Example 1 Culture of the transformants and the preparation of the culture supernatant

The transformed cell L-IS11 IgE-9 (IFO 50056, KFCC-10280) obtained in Reference Example 1 was inoculated to 250 ml of Eagle's MEM medium (Nissui Seiyaku, Japan) containing 25mM of N-2-hydroxyethyl pyperadine-N'-2-ethane sulfonic acid and 10% fetal calf serum in a Falcon roller bottle 3027 (Becton Dickinson, USA) to be 2 × 10⁷/ml of cell concentration. It was cultured for three or four days with revolving the bottle once/minute at 37°C. Then culture was twice conducted at 37°C, for three days, in the medium where the serum concentration had been lowered to 2%. The culture product was centrifugated (J.S-4.2-rotar; 1,500 rotations/minute; for five minutes) by Beckman centrifuge J-6B to obtain 750 ml of the culture supernatant comprising an Fc fragment.

Example 2 Preparation of substantially pure Fc fragment

(1) Salting out with ammonium sulfate
Salting-out with ammonium sulfate (saturated with 40-60% of ammonium sulfate) of 20 liters of the culture supernatant obtained according to the method of Example 1 was carried out at 5°C, the produced precipitate was isolated by centrifugation (J.S-4.2 rotar; 4,000 rotations/minute; for 60 minutes) with Beckman centrifuge J-6B. The precipitate was solubilized into 650 ml of phosphate-buffered saline (PBS; 8.1mM NaH$_2$PO$_4$, 1.5mM KH$_2$PO$_4$, 2.7 mM KCl, 137 mM NaCl, pH 7.4), followed by dialysis against 10 liters of the same buffer for 40 hours at 5°C to remove ammonium sulfate in the solution. The buffer was once replaced in the middle of the procedure.

(2) Immunoaffinity chromatography
E235I63-Affi Gel 10 column (1.5 × 6 cm) prepared in Reference Example 2 was equilibrated with PBS and 750 ml of dialyzed solution obtained in (1) was applied to the column. Then, the column was washed with 230 ml of the same buffer, 20 ml of PBS containing 0.5 M NaCl, 20 ml of 0.2M sodium acetate buffer (pH 7.2) containing 0.15 M NaCl, 80 ml of 0.2M sodium acetate buffer (pH 7.2) containing 0.15 M NaCl and 3M urea, 40 ml of 0.2 M sodium acetate buffer (pH 6.0) containing 0.15 M NaCl and 3 M urea, 30 ml of 0.2 M sodium acetate buffer (pH 5.0) containing 0.15 M NaCl and 3M urea, and 50 ml of PBS in turn. Fifty ml of the active fraction eluted with the buffers of pH 6.0 and pH 5.0 had been dialyzed against 1 liter of PBS at 5°C for 3 days. The buffer was twice replaced in the middle of the procedure. The dialyzed solution was centrifuged (JA-17 rotar; 15,000 rotations/ minute; for 60 minutes) by Beckman centrifuge J-21 to obtain 52 ml of centrifuged supernatant.

(3) Gel filtration chromatography using Sephacryl S-200
20 ml, a part of the centrifuged supernatant obtained in (2) was concentrated to 1.5ml by YM-5 membrane (Amicon, U.S.A.) The whole obtained concentrated solution was applied to Sephacryl S-200 column (1.6 × 115cm) previously equilibrated with PBS and an Fc fragment was eluted with the same buffer. Each 20 μl of active fractions was applied to SDS-polyacrylamide gel electrophoresis and only fractions containing no aggregates were collected to obtain 12.5 ml of solution containing 1.18 mg (1.1 × 10$^6$ units) of substantially pure Fc fragment.

Example 3 Desalt by High Performance Liquid Chromatography using reverse-column
Six μl of trifluoroacetic acid was added to 3 ml of the solution containing 282 μg of substantially pure Fc fragment obtained in Example 2 (3) and the mixture was filtrated by membrane-filter (Ekicrodisk; Gelman Science Japan). 2.5 ml from the filtrate was adsorbed to AP202 300Å(C8) reverse phased column (YMC Shimakyu) and high performance liquid chromatography using trifluoroacetic acid-acetonitrile as elution solvent was conducted in the following conditions:.
Column: AP 202 300Å (C8) (4.6 × 150mm), Column temperature: 30°C; Elution solvent A: 0.1% trifluoroacetic acid-99.9% water; Elution solvent B: 0.1% trifluoroacetic acid-99.9% acetonitrile; Elution program: 0 minute (80% A + 20% B)- 30 minutes (20% A + 80% B) - 35 minutes (20% A + 80% B) - 35.1 minutes (80% A + 20 % B), Elution speed : 1 ml/min.; Detection wave length : 280 nm; Loop: 3 ml. Under these conditions, an Fc fragment was eluted at the position of approximately 19 minute retention time.
The substantially pure Fc fragment obtained in the above-mentioned procedure has following properties:

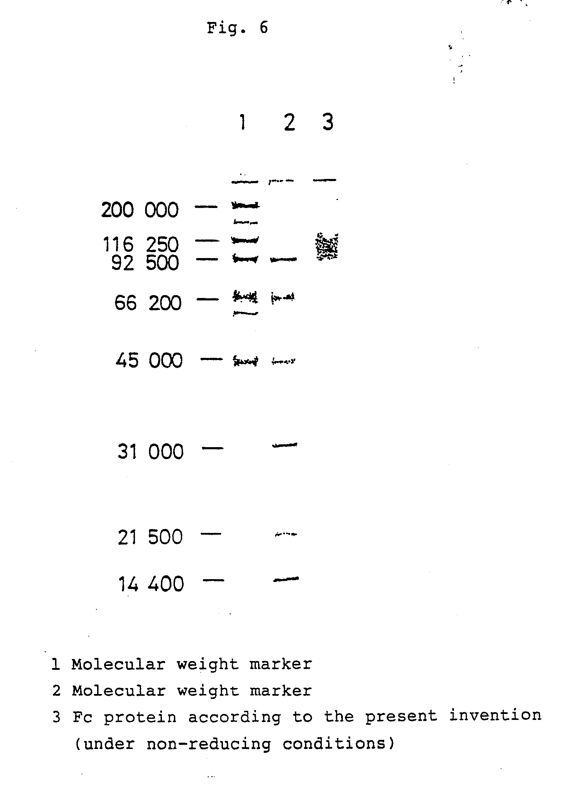

(1) Homogeneity:
Purity of the Fc fragment obtained in Example 2 was examined by SDS-polyacrylamide slab gel electrophoresis according to Laemmli's method [Nature, 227 680(1970)]. As the result, the Fc fragment showed a wide band (See Fig. 6).

(2) Molecular weight:
Molecular weight of the Fc fragment was calculated as 110,000 ± 10,000 dalton under non-reducing conditions and as 55,000 ± 5,000 dalton under reducing conditions from the result of SDS-polyacrylamide slab gel electrophoresis.

(3) Amino acid composition:
19 μg of Fc-fragment obtained in Example 3 was placed in a glass tube for hydrolysis. Then, constant-boiling HCl which contains 4% thioglycolic acid was added into the test tube and sealed in vacuo. Hydrolysis was carried out at 110°C for 24 hours, 48 hours and 72 hours respectively. After hydrolysis, the tubes were opened and the hydrochloric acid was removed under reduced pressure and the residues were dissolved in 0.02N HCl to be conducted by amino acid analysis using 835 type-amino acid analyzer (Hitachi, Japan). Regarding cystine and cysteine, the Fc-fragment was oxidized by performic acid, followed by hydrolysis for 24 hours in constant-boiling HCl under reduced pressure, they were quantitatively analyzed as cysteic acid by amino acid analyzer.

The amino acid analytical values were obtained by taking an average of the values obtained in hydrolysis for 24, 48 and 72 hours. The value for serine and threonine were obtained by extrapolation of hydrolysis time to 0 hour. The results are shown in Table 1.

Table 1

| Amino acid | Mole % |
|---|---|
| Asp/Asn | 7.5 |
| Thr | 11.5 |
| Ser | 9.3 |
| Glu/Gln | 11.0 |
| Pro | 6.4 |
| Gly | 6.0 |
| Ala | 6.1 |
| 1/2 Cys | 2.3 |
| Val | 6.4 |
| Met | 1.4 |
| Ile | 3.0 |
| Leu | 7.9 |
| Tyr | 2.0 |
| Phe | 3.6 |
| Lys | 5.0 |
| His | 2.8 |
| Arg | 6.0 |
| Trp | 1.9 |

(4) Amino Terminal Amino Acid Sequence:

76 µg of the Fc fragment obtained in Example 3 was subjected to an automated Edman degradation using a gas-phase protein sequencer (model 470A manufactured by Applied Biosystem Inc.) to analyze the amino terminal amino acid sequence. The yielded phenylthiohydantoin-amino acids (PTH-amino acids) were identified by means of high performance liquid chromatography using a Micropack SP-ODS column (manufactured by Varian Inc.). PTH-amino acids detected at each step were shown in Table 2.

## Table 2

| Step | Detected PTH-Amino acid |
|------|-------------------------|
| 1 | Alanine |
| 2 | Proline |
| 3 | Threonine |
| 4 | Serine |
| 5 | X* |
| 6 | Serine |
| 7 | Threonine |
| 8 | Lysine |
| 9 | Lysine |

* : Unidentified

(5) COOH Terminal Amino Acid

190 μg of the Fc fragment obtained in Example 3 was placed in a glass tube for hydrazinolysis. Then, 0.2 ml of hydrazine anhydride was added into the test tube and sealed under reduced pressure, followed by heating at 100°C for six hours. The obtained hydrazinolysate was dried to solid using speed back concentrator (Servant, U.S.A.) and was solubilized into distilled water. Benzaldehyde was added to the solution and the mixture was shaken vigorously at room temperature for 1 hour followed by centrifugation to obtain the supernatant. The supernatant was dried to solid and it was applied to amino acid analyzer with 835 model amino acid analyzer by Hitachi. As the result, 2.1 nmole of lysine was detected.

(6) Neutral Sugar:

19.5 μg of the Fc-fragment obtained in Example 3 was placed in a glass tube. After the removal of the solvent under the reduced pressure, neutral sugar was quantitatively analyzed according to phenol-sulfuric acid method (Analytical Chemistry volume 28, p.350, 1956) using mannose as a standard. As the result, 22 moles of sugar per 1 mole of Fc-fragment was detected in calculating the molecular weight of Fc-fragment (dimer) as 76822 dalton.

(7) Sialic Acid:

97.5 μg of the Fc-fragment was placed in a glass tube. After the removal of the solvent under the reduced pressure, sialic acid was quantitatively analyzed according to periodic acid-resorcinol method (J. Biol. Chem. 246, 430/1971) using N-acetylneuraminic acid as a standard. As the result, 4 moles of sialic acid for 1 mole of the Fc-fragment (dimer).

**Claims**

(1) A highly purified fused protein which comprises a first protein having the amino acid sequence: Ala-Pro-Thr-Ser-Ser-Ser-Thr-Lys-Lys-Thr-Gln-Arg-Asn-Ser-Met-Leu and a human IgE Fc fragment joined to the C-terminus of the first protein.

(2) The fused protein of claim 1, which is a glycosylated protein comprising sugar and which has a specific activity of at least $5 \times 10^5$ units/mg and a molecular weight, based on SDS-polyacrylamide gel electrophoresis of 110,000 ± 10,000 dalton under non-reducing conditions and 55,000 ± 5000 dalton under reducing conditions.

0 269 455

(3) A production method for a highly purified fused protein which comprises cultivating in a medium an animal cell capable of secreting a fused protein which comprises a first protein having the amino acid sequence:

Ala-Pro-Thr-Ser- Ser-Ser-Thr-Lys-Lys-Thr-Gln-Arg-Asn-Ser-Met-Leu and a human IgE Fc fragment joined to the C-terminus of the first protein, and collecting the protein accumulated in the medium by the procedure comprising immunoaffinity chromatography.

(4) A method of claim 3 wherein the fused protein is a glycosylated protein comprising sugar and has a specific activity of at least $5 \times 10^5$ units/mg and a molecular weight, based on SDS-polyacrylamide gel electrophoresis of 110,000 $\pm$ 10,000 dalton under non-reducing conditions and 55,000 $\pm$ 5000 dalton under reducing conditions.

(5) A method of claim 3 or claim 4, wherein the procedure comprising immunoaffinity chromatography further comprises ammonium sulfate salting out or gel filtration chromatography, or combination thereof.

(6) A method of claim 5, wherein the procedure is the combination of ammonium sulfate salting out, immunoaffinity chromatography and gel filtration chromatography in this order.

(7) A method of claim 3, 4, 5 or 6, wherein the immunoaffinity chromatography is carried out by using monoclonal antibody recognizing Fc portion as a ligand.

(8) A method of claim 7, wherein the monoclonal antibody is E235I63 or E120.02.

(9) The use of a highly purified fused protein as defined in claim 1 for the production of a medicine for allergy.

11

0269455

Fig. 1

Fig. 2

0269455

Fig. 3-1

5'
GGGGGGGGGGGGGGGGGGGGATCACTCTCTTTAATCACTACTCACAGTAACC
Pst I

TCAACTCCTGCCACA ATG TAC AGG ATG CAA CTC CTG TCT TGC

ATT GCA CTA AGT CTT GCA CTT GTC ACA AAC AGT GCA CCT
Hgi AI

ACT TCA AGT TCT ACA AAG AAA ACA CAG CTA CAA CTG GAG
Alu I

CAT TTA CTG CTG GAT TTA CAG ATG ATT TTG AAT GGA ATT

AAT AAT TAC AAG AAT CCC AAA CTC ACC AGG ATG CTC ACA

TTT AAG TTT TAC ATG CCC AAG AAG GCC ACA GAA CTG AAA

CAT CTT CAG TGT CTA GAA GAA GAA CTC AAA CCT CTG GAG

Fig. 3-2

GAA GTG CTA AAT TT<u>A GCT</u> CAA AGC AAA AAC TTT CAC <sup>80</sup>TTA
　　　　　　　　　　　　　　Alu I

AGA CCC AGG GAC TTA ATC AGC AAT ATC AAC GTA ATA GTT

<sup>100</sup>
CTG GAA CTA AAG GGA TCT GAA ACA ACA TTC ATG TGT GAA

TAT GCT GAT GAG ACA GCA ACC ATT GTA GAA TTT CTG AAC

120
AGA TGG ATT ACC TTT TGT CAA AGC ATC ATC TCA ACA CTG

133
ACT TGA TAATTAAGTGCTTCCACTTAAAACATATC<u>AGGCCT</u>TCTATTT
　　　　　　　　　　　　　　　　　　　　　　　　　　　Stu I

ATTTAAATATTTAAATTTTACCCCCCCCCCCCCCC<sup>3'</sup>

0269455

Fig. 4

## F i g. 5 − 1

I L − 2　leader sequence

　-Met-Tyr-Arg-Met-Gln-Leu-Leu-Ser-Cys-Ile-Ala-Leu-Ser-Leu-Ala-Leu-Val-Thr-Asn-Ser

I L − 2　N terminal 11 amino acids residues

　-Ala-Pro-Thr-Ser-Ser-Ser-Thr-Lys-Lys-Thr-Gln

L inker

　-Arg-Asn-Ser-Met-Leu

Fc portion of I g E

　　　　　　　　　226　　　　　　　　　　　　　　　　　　　　　　　　　　240
　　　　　　　　　-Asp-Asn-Lys-Thr-Phe-Ser-Val-Cys-Ser-Arg-Asp-Phe-Thr-Pro-Pro

　-Thr-Val-Lys-Ile-Leu-Gln-Ser-Ser-Cys-Asp-Gly-Gly-Gly-His-Phe-Pro-Pro-Thr-Ile-Gln

　-Leu-Leu-Cys-Leu-Val-Ser-Gly-Tyr-Thr-Pro-Gly-Thr-Ile-Asn-Ile-Thr-Trp-Leu-Glu-Asp

　-Gly-Gln-Val-Met-Asp-Val-Asp-Leu-Ser-Thr-Ala-Ser-Thr-Thr-Gln-Glu-Gly-Glu-Leu-Ala

　-Ser-Thr-Gln-Ser-Glu-Leu-Thr-Leu-Ser-Gln-Lys-His-Trp-Leu-Ser-Asp-Arg-Thr-Tyr-Thr

　-Cys-Gln-Val-Thr-Tyr-Gln-Gly-His-Thr-Phe-GIu-Asp-Ser-Thr-Lys-Lys-Cys-Ala-Asp-Ser

　-Asn-Pro-Arg-Gly-Val-Ser-Ala-Tyr-Leu-Ser-Arg-Pro-Ser-Pro-Phe-Asp-Leu-Phe-Ile-Arg

Fig. 5-2

-Lys-Ser-Pro-Thr-Ile-Thr-Cys-Leu-Val-Val-Asp-Leu-Ala-Pro-Ser-Lys-Gly-Thr-Val-Asn

-Leu-Thr-Trp-Ser-Arg-Ala-Ser-Gly-Lys-Pro-Val-Asn-His-Ser-Thr-Arg-Lys-Glu-Glu-Lys

-Gln-Arg-Asn-Gly-Thr-Leu-Thr-Val-Thr-Ser-Thr-Leu-Pro-Val-Gly-Thr-Arg-Asp-Trp-Ile

-Glu-Gly-Glu-Thr-Tyr-Gln-Cys-Arg-Val-Thr-His-Pro-His-Leu-Pro-Arg-Ala-Leu-Met-Arg

-Ser-Thr-Thr-Lys-Thr-Ser-Gly-Pro-Arg-Ala-Ala-Pro-Glu-Val-Tyr-Ala-Phe-Ala-Thr-Pro

-Glu-Trp-Pro-Gly-Ser-Arg-Asp-Lys-Arg-Thr-Leu-Ala-Cys-Leu-Ile-Gln-Asn-Phe-Met-Pro

-Glu-Asp-Ile-Ser-Val-Gln-Trp-Leu-His-Asn-Glu-Val-Gln-Leu-Pro-Asp-Ala-Arg-His-Ser

-Thr-Thr-Gln-Pro-Arg-Lys-Thr-Lys-Gly-Ser-Gly-Phe-Phe-Val-Phe-Ser-Arg-Leu-Glu-Val

-Thr-Arg-Ala-Glu-Trp-Glu-Gln-Lys-Asp-Glu-Phe-Ile-Cys-Arg-Ala-Val-His-Glu-Ala-Ala

551                                                                       556
-Ser-Pro-Ser-Gln-Thr-Val-Gln-Arg-Ala-Val-Ser-Val-Asn-Pro-Gly-LysOH

0269455

Fig. 6

1    2    3

200 000 —

116 250 —
92 500 —

66 200 —

45 000 —

31 000 —

21 500 —

14 400 —

1 Molecular weight marker
2 Molecular weight marker
3 Fc protein according to the present invention
  (under non-reducing conditions)